**Europäisches Patentamt**

**European Patent Office**

**Office européen des brevets**

(19)

(11) Veröffentlichungsnummer : **0 151 201**
**B1**

# (12) EUROPÄISCHE PATENTSCHRIFT

(45) Veröffentlichungstag der Patentschrift :
**12.11.86**

(21) Anmeldenummer : **84101241.2**

(22) Anmeldetag : **07.02.84**

(51) Int. Cl.⁴ : **A 61 K 33/34**, A 61 K 7/04

(54) Keratolytisches Mittel, geeignet zum Entfernen von Warzen.

(43) Veröffentlichungstag der Anmeldung :
**14.08.85 Patentblatt 85/33**

(45) Bekanntmachung des Hinweises auf die Patenterteilung : **12.11.86 Patentblatt 86/46**

(84) Benannte Vertragsstaaten :
**BE DE FR GB IT NL**

(56) Entgegenhaltungen :
EP-A- 0 026 532
FR-A- 2 310 768
**F. WINTER: "Handbuch der gesamten Parfumerie und Kosmetik", 1927, Seiten 917-918, Julius Springer, Wien, AT;**
**SOAP & CHEMICAL SPECIALTIES, Band 47, Nr. 6, Juni 1971, Seiten 31-32, McNair-Dorland Co., New York, USA; H. GOLDSCHMIEDT: "Cosmetics for foot care"**
**CHEMICAL ABSTRACTS, Band 99, Nr. 8, August 1983, Seite 300, Nr. 58746h, Columbus, Ohio, USA**
**Roempp's Chemielexikon Stichwort "Vaseline"**

(73) Patentinhaber : **Pestelic, Zvonko**
**Thermaeniusgatan 90**
**S-64 400 Torshälla (SE)**

(72) Erfinder : **Pestelic, Zvonko**
**Thermaeniusgatan 90**
**S-64 400 Torshälla (SE)**

(74) Vertreter : **Popp, Eugen, Dr. et al**
**MEISSNER, BOLTE & PARTNER Postfach 86 06 24**
**D-8000 München 86 (DE)**

## Beschreibung

Die Erfindung bezieht sich auf ein keratolytisches Mittel zum Entfernen von Warzen, Hornhaut, Hühneraugen und Schwielen. Es ist besonders hervorragend zur dauerhaften Entfernung von Warzen geeignet.

Zur Behandlung von Warzen sind neben der chirurgischen Entfernung dieser Wucherungen eine Vielzahl von Salben, Pudern, Stiften und Tinkturen bekannt. Diese Produkte enthalten vielfach Salicylsäure als Einzelwirkstoff oder in Kombination mit anderen Arzneistoffen (vgl. Rote Liste 1983, *31*, 419). Eine dauerhafte Entfernung der Warzen wird allerdings mit keinem der bekannten Mittel erreicht. Die Warzen kommen immer wieder, die Beschwerden und die kosmetischen Probleme tauchen erneut auf und machen eine neuerliche, meist langwierige Behandlung notwendig, die wieder nur — wenn überhaupt — kurze Zeit Erfolg hat.

Hier will die Erfindung Abhilfe schaffen. Die Erfindung, wie sie in den Ansprüchen gekennzeichnet ist, löst die Aufgabe, ein Mittel zum dauerhaften Entfernen von Warzen bereitzustellen.

Das erfindungsgemäße keratolytische Mittel liegt bevorzugt in Form einer Salbe vor, jedoch sind auch andere übliche Konfektionierungsmöglichkeiten wie Tinkturen und Stifte möglich. Insbesondere kann das Mittel neben der Salbenform auch in Form einer Tinktur vorliegen, in der die angegebenen Bestandteile gelöst bzw. aufgeschlämmt und/oder dispergiert sind. Als Lösungsmittel kommen übliche Lösungsmittel wie z. B. Ethanol und Aceton in Frage.

Erfindungsgemäß besteht das Mittel aus den Wirkstoffen

— Vaseline, insbesondere Weißvaseline und/oder Borvaseline
     — Kupfernitrat in Pulverform
     — gelbem Schwefel in Pulverform
     — Salicylsäure.

Kern der Erfindung ist die Kombination der genannten Bestandteile. Die Vorteile der Erfindung sind im wesentlichen darin zu sehen, daß auf einfachste Art eine dauerhafte Entfernung der Warzen ermöglicht wird.

Die erfindungsgemäße Kombination hat bei vielen Patienten, die erfolglos mit bekannten Präparaten behandelt wurden, überraschend zu einer endgültigen Heilung geführt.

Als besonders wirksam haben sich die homogenen Mischungen aus

40-60 % Vaseline,
20-45 % pulverförmigem Kupfernitrat
   8-22 % gelbem pulverförmigem Schwefel und
0,5-10 % Salicylsäure erwiesen.

Besonders günstige Anteile der Vaseline liegen bei 48-54 % und insbesondere 50 % des Mittels bzw. der Salbe. Die Vaseline kann teilweise oder gänzlich aus Weißvaseline oder Borvaseline bestehen. Als günstiger Borsäuregehalt der Borvaseline haben sich bis zu 10 % und insbesondere bis zu 3 % erwiesen.

Es wird bevorzugt das handelsübliche Kupfer-(II)-nitrat verwendet.

Die Mengen liegen vorteilhaft bei 26-45 % und insbesondere 30 % des Mittels bzw. der Salbe.

Als Schwefel kann handelsübliches gelbes Schwefelpulver in Mengen von besonders 12-18 % und vorzugsweise 15 % bezogen auf das Mittel bzw. Salbenpräparat eingesetzt werden.

Die Salicylsäure wird in dem erfindungsgemäßen Mittel bzw. der Salbe besonders günstig in Mengen von 4-7 % und bevorzugt 5 % verwendet.

Die vorstehenden Prozentangaben beziehen sich auf das Gewicht der Summe der Bestandteile : Vaseline (geschütztes Warenzeichen), Schwefel, Salicylsäure, Kupfernitrat.

Beispiel

Es wurden eine Salbe zu bereitet aus :

50 % Vaseline
30 % pulverförmiges Kupfernitrat
15 % pulverförmiger gelber Schwefel
  5 % Salicylsäure.

## Patentansprüche

1. Keratolytisches Mittel, geeignet zum Entfernen von Warzen, dadurch gekennzeichnet, daß es als Wirkstoff enthält :

40-60 % Vaseline,
20-45 % und insbesondere 20-40 % pulverförmiges Kupfernitrat
  8-22 % pulverförmigen gelben Schwefel
0,5-10 % Salicylsäure.

2. Keratolytisches Mittel gemäß Anspruch 1, dadurch gekennzeichnet, daß es enthält :

48-54 % Vaseline,
26-45 % pulverförmiges Kupfernitrat
12-18 % pulverförmigen gelben Schwefel
  4- 7 % Salicylsäure.

3. Keratolytisches Mittel gemäß Anspruch 1 oder 2, dadurch gekennzeichnet, daß es besteht aus :

50 % Vaseline,
30 % pulverförmiges Kupfernitrat
15 % pulverförmigen gelben Schwefel
  5 % Salicylsäure.

4. Keratolytisches Mittel gemäß einem der Ansprüche 1 bis 3, dadurch gekennzeichnet, daß die Vaseline Borvaseline und/oder Weißvaseline ist.

5. Keratolytisches Mittel gemäß einem der Ansprüche 1 bis 4, dadurch gekennzeichnet, daß die Borvaseline maximal 10 % Borsäure enthält.

6. Keratolytisches Mittel gemäß einem der Ansprüche 1 bis 4, dadurch gekennzeichnet, daß der Borsäuregehalt der Borvaseline maximal 3 % beträgt.

7. Keratolytisches Mittel gemäß einem der Ansprüche 1 bis 6, dadurch gekennzeichnet, daß es in Form einer Salbe vorliegt.

## Claims

1. Keratolytic agent, suitable for removing warts, characterised in that as active ingredients it contains :

40-60 % vaseline,
20-45 % and in particular 20-40 % copper nitrate in the form of powder
8-22 % yellow sulphur in the form of powder
0.5-10 % salicylic acid.

2. Keratolytic agent according to Claim 1, characterised in that it contains :

48-54 % vaseline,
26-45 % copper nitrate in the form of powder
12-18 % yellow sulphur in the form of powder
4-7 % salicylic acid.

3. Keratolytic agent according to Claim 1 or 2, characterised in that it consists of

50 % vaseline,
30 % copper nitrate in the form of powder
15 % yellow sulphur in the form of powder
5 % salicylic acid.

4. Keratolytic agent according to one of Claims 1 to 3, characterised in that the vaseline is boric acid vaseline and/or white vaseline.

5. Keratolytic agent according to one of Claims 1 to 4, characterised in that the boric acid vaseline contains a maximum of 10 % boric acid.

6. Keratolytic agent according to one of Claims 1 to 4, characterised in that the boric acid content of the boric acid vaseline amounts to 3 % maximum.

7. Keratolytic agent according to one of Claims 1 to 6, characterised in that it is in the form of an ointment.

## Revendications

1. Composition kératolytique, apte à enlever des verrues, caractérisée en ce qu'elle contient, comme principe actif :

de 40 à 60 % de vaseline,
de 20 à 45 %, et notamment de 20 à 40 % de nitrate de cuivre sous forme de poudre,
de 8 à 22 % de soufre jaune sous forme de poudre,
de 0,5 à 10 % d'acide salicylique.

2. Composition kératolytique suivant la revendication 1, caractérisée en ce qu'elle contient :

de 48 à 54 % de vaseline,
de 26 à 45 % de nitrate de cuivre sous forme de poudre,
de 12 à 18 % de soufre jaune sous forme de poudre,
de 4 à 7 % d'acide salicylique.

3. Composition kératolytique suivant la revendication 1 ou 2, caractérisée en ce qu'elle est constituée :

de 50 % de vaseline,
de 30 % de nitrate de cuivre sous forme de poudre,
de 15 % de soufre jaune sous forme de poudre,
de 5 % d'acide salicylique.

4. Composition kératolytique suivant l'une des revendications 1 à 3, caractérisée en ce que la vaseline est de la vaseline de bore et/ou de la vaseline blanche.

5. Composition kératolytique suivant l'une des revendications 1 à 4, caractérisée en ce que la vaseline de bore contient, au maximum, 10 % d'acide borique.

6. Composition kératolytique suivant l'une des revendications 1 à 4, caractérisée en ce que la teneur en acide borique de la vaseline de bore est de 3 % au maximum.

7. Composition kératolytique suivant l'une des revendications 1 à 6, caractérisée en ce qu'elle se présente sous la forme d'une pommade.